Europäisches Patentamt

European Patent Office

Office européen des brevets

(11) Veröffentlichungsnummer: **0 432 567 A1**

## EUROPÄISCHE PATENTANMELDUNG

(21) Anmeldenummer: **90122610.0**

(51) Int. Cl.⁵ **A61F 13/04**

(22) Anmeldetag: **27.11.90**

(30) Priorität: **15.12.89 DE 8914749 U**

(43) Veröffentlichungstag der Anmeldung:
**19.06.91 Patentblatt 91/25**

(84) Benannte Vertragsstaaten:
**AT BE DE ES FR GB IT NL SE**

(71) Anmelder: **Beiersdorf Aktiengesellschaft**
**Unnastrasse 48**
**W-2000 Hamburg 20(DE)**

(72) Erfinder: **Lenzen, Franz Richard**
**Sollbrüggenstrasse 58**
**W-4150 Krefeld(DE)**

(54) **Gehgipssohle.**

(57) Gehgipssohle mit einem Sohlenkörper, wobei in der Gehgipssohle wenigstens zwei Durchtritte zur Querführung eines darin eingezogenen Gurtbandsystems vorgesehen sind, die dadurch gekennzeichnet ist, daß das Gurtbandsystem nur ein einziges Gurtband (4) aufweist, das die Durchtritte (8, 9) quer durchläuft, wobei jedoch die Gurtbandabschnitte (22, 23) außerhalb der Durchtritte (8, 9) und zwischen dem vorderen und hinteren Durchtritt (8, 9) diagonal und sich kreuzend von einer Seite zur anderen verlaufen und die beiden Enden außerhalb der Durchtritte (8, 9) über ein Verbindungsmittel (24) verstellbar gekuppelt sind.

EP 0 432 567 A1

## GEHGIPSSOHLE

Die Erfindung betrifft eine Gehgipssohle mit einem Sohlenkörper, wobei in der Gehgipssohle wenigstens zwei Durchtritte zur Querführung eines darin eingezogenen Gurtbandsystems vorgesehen sind. Solche Gehgipssohlen werden unter einem Gehgips - oder auch unter einem Liegegips - angeschnallt und haben den Vorteil, daß der Gehgips selbst nicht verschmutzt und daß ein besserer Abrollen erreicht wird.

Es sind verschiedene Ausführungsformen solcher Gehgipssohlen bekannt. So ist beispielsweise in der DE-PS 922 010 eine Gehgipssohle offenbart, die allein aus einem Gummisohlenkörper besteht. Im vorderen Bereich ist ein Gurtbandsystem zum Anschnallen des Gummisohlenkörpers vorgesehen. Im mittleren und hinteren Bereich des Sohlenkörpers sind untenseitig Nuten eingeformt, die sich teilweise kreuzen. In diese Nuten können Mullbinden oder dergleichen eingelegt werden, die bis zu dem Gipsverband heraufgeführt und in diesen eingegipst werden. Nachteilig ist dabei, daß diese Gehgipssohle nicht abgenommen werden kann, es sei denn, man schneidet die Mullbinden durch und befestigt die Gehgipssohle anschließend mit neuen Mullbinden, die dann wieder eingegipst werden müssen.

In der CH-PS 293 292 ist eine Gehgipssohle beschrieben, die einen nach unten gewölbten Sohlenkörper aufweist, der zwei hintereinanderliegende Durchtritte hat. Durch diese Durchtritte ist jeweils ein Gurtband zur Befestigung der Gehgipssohle am Gehgips eingezogen. Damit die Gehgipssohle nicht vom Gehgips abrutscht, ist zusätzlich in den Gehgips ein Bügelträger eingegipst, der nach unten vorsteht und Aufnahme in eine daran angepaßte Nut im Sohlenkörper findet. Nur mit diesem Bügelträger liegt der Fuß des Patienten auf der Gehgipssohle auf. Nachteilig ist, daß der Bügelträger sehr genau eingegipst werden muß, damit die Gehgipssohle anschließend die richtige Stellung zum Fuß hat. Außerdem ist der Sohlenkörper sehr voluminös, was zu einer starken Verlängerung des geschienten Beins und damit zu einer starken Gehbehinderung führt.

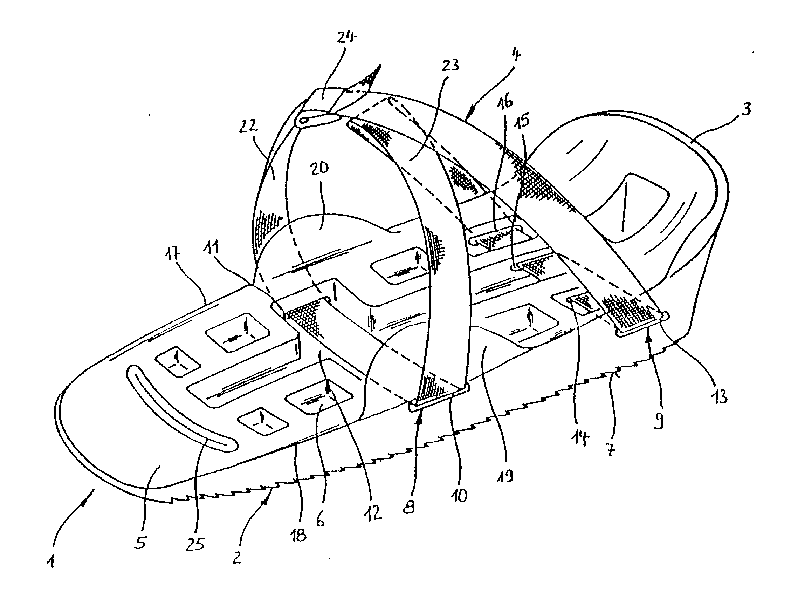

In "Zeitschrift für Orthopädie und ihre Grenzgebiete", 1968, Band 104, Seiten 109 bis 116 sind verschiende Ausführungen von Gehgipssohlen dargestellt. In Abbildung 7c ist eine Gehgipssohle offenbart, die am hinteren Ende eine Fersenstütze aufweist. Sowohl an der Fersenstütze als auch im vorderen Bereich des Sohlenkörpers sind Gurtbänder angeordnet, mit denen die Gehgipssohle an einen Gehgips angeschnallt werden kann. Da die Auflagefläche des Sohlenkörpers nahezu eben ist, hat man trotz des vorderen Gurtbandes einen schlechten Halt in diesem Bereich. Auch das hintere Gurtband verläuft nicht optimal, da es den Gehgips im Fersenbereich wegen seines im wesentlichen horizontalen Verlaufes nicht am Abheben vom Sohlenkörper hindert.

Schließlich ist in der DE-PS 27 40 400 eine Gehgipssohle mit einem Sohlenkörper und einer hinten angeformten Fersenstütze offenbart, die sich dadurch auszeichnet, daß Durchtritte im vorderen Bereich und in der Fersenstütze vorgesehen sind, in die jeweils ein Gurtband eingezogen ist. Außerdem sind die Ränder des Sohlenkörpers vorragend ausgeführt. Durch diese Ränder soll erreicht werden, daß der Fuß im Bereich des Sohlenkörpers einwandfrei festgelegt wird. Das an der Fersenstütze in Richtung derselben verlaufende Gurtband führt zu einer starken Erhöhung der Festigkeit der Stütze in diesem Bereich, wodurch Materialeinsparungen möglich sind. Nachteilig ist auch hier die Gurtbandführung. Das hintere Gurtband kann wegen seiner im wesentlichen waagerechten Führung nicht ein Abheben des Fersenbereichs des Gehgipses verhindern. Außerdem muß die seitliche Führung im wesentlichen von der Fersenstütze aufgenommen werden. Auch im vorderen Bereich ist die Festlegung des Gehgipses nicht optimal.

Der Erfindung liegt die Aufgabe zugrunde, eine Gehgipssohle der eingangs genannten Art so zu gestalten, daß die Gehgipssohle wesentlich besser am Gehgips festgelegt wird.

Diese Aufgabe wird erfindungsgemäß dadurch gelöst, daß das Gurtbandsystem nur ein einziges Gurtband aufweist, das die Durchtritte quer durchläuft, wobei jedoch die Gurtbandabschnitte außerhalb der Durchtritte zwischen dem vorderen und hinteren Durchtritt diagonal und sich kreuzend von einer Seite zur anderen verlaufen und die beiden Enden außerhalb der Durchtritte über ein verstellbares Verbindungsmittel gekuppelt sind.

Diese diagonale Überkreuzführung des einzigen Gurtbandes oberhalb des Sohlenkörpers hat zu einer wesentlich besseren Festlegung der Gehgipssohle geführt. Dies hängt offenbar damit zusammen, daß die über den Gehgips laufenden Abschnitte des Gurtbandes wesentlich länger sind als bei den bekannten Gurtbandsystemen mit zwei Gurtbändern und daß sie erheblich größere Anteile haben, mit denen sie an den Seiten des Gehgipses verlaufen. Hinzu kommt, daß die Gehgipssohle in der Handhabung wesentlich einfacher und sicherer ist. Durch Zug an dem aus den einzigen Verbindungsmittel herausragenden Ende wird das Gurtband gleichmäßig über seine gesamte Länge an den Gehgips herangeführt und mit diesem verspannt. Es kann also nicht mehr - wie im Stand der Technik - passieren, daß das eine Gurtband zu lose und das andere zu fest angezogen wird. Für die Krankenhausträger ergibt sich der Vorteil, daß nur ein Gurtband vorgehalten und eingezogen werden muß.

Als Verbindungsmittel kommen insbesondere Klemmschnallen und Klettverschlüsse in Frage, aber auch einfache Schnallen mit Schnallstiften.

Von besonderem Vorteil ist, wenn die Gehgipssohle in an sich bekannter Weise hintenseitig eine nach oben vorstehende Fersenstütze aufweist. Zwar kann dann auch der hintere Durchtritt durch die Fersenstütze verlaufen, wie dies bei der Gehgipssohle nach der DE-PS 27 40 400 vorgesehen ist. Als vorteilhafter hat sich jedoch erwiesen, daß der hintere Durchtritt vor der Fersenstütze angeordnet ist, weil dann die zu diesem Durchtritt hinlaufenden Gurtbandabschnitte einerseits eine bessere seitliche Festlegung gewährleisten und andererseits auch einen Zug nach unten mit entsprechend besserer vertikaler Festlegung bewirken können.

Eine weitere Verbesserung der Festlegung der Gehgipssohle am Gehgips ergibt sich dann, wenn die Gehgipssohle vorragende Seitenränder aufweist, weil hierdurch die von dem Gurtband bewirkte seitliche Führung am Gehgips unterstützt wird. Insoweit kann jedoch die seitliche Führung noch dadurch verbessert werden, daß von den Seitenrändern im Bereich des vorderen Durchtritts vorzugsweise mindestens 2,5 cm hohe Führungsstege hochstehen, die flexibel ausgebildet sind und über dessen Außenseiten das Gurtband verläuft. Hierdurch wird ein seitliche Herausrutschen des Gehgipses zuverlässig verhindert. Beim Strammziehen des Gurtbandes werden die flexiblen Führungsstege an dem Gehgips angepreßt und bilden einen zusätzlichen Halt. Darüberhinaus erlaubt die Flexibilität der Führungsstege auch die Aufnahme von Gehgipsen, die etwas breiter sind als der Abstand der Führungsstege. Die Führungsstege können dann nach außen ausweichen und werden beim Strammziehen des Gurtbandes wieder an den Gehgips angepreßt. Insgesamt ergibt sich hierdurch eine deutliche Verbesserung in der Festlegung der Gehgipssohle am Gehgips, und zwar auch dann, wenn der Gehgips überbreite hat.

Die Führungsstege sind zweckmäßigerweise an dem Sohlenkörper angeformt, bestehen also aus dessen Material. Damit sie sich der Krümmung des Gehgipses besser anpassen, sollte die Flexibilität der Führungsstege nach oben hin zunehmen. Dies wird zweckmäßigerweise dadurch erreicht, daß die Erstreckung der Führungsstege in Längsrichtung und/oder deren Dicke nach oben hin abnehmen. Die Führungsstege haben vorteilhafterweise in etwa eine Halbkreisform.

In der Zeichnung ist die Erfindung an Hand eines perspektivisch dargestellten Ausführungsbeispiels näher veranschaulicht. Sie zeigt die erfindungsgemäße Gehgipssohle (1), die im wesentlichen aus einem Sohlenkörper (2), einer hintenseitig vom Sohlenkörper (2) hochstehenden Fersenstütze (3) und einem Gurtband (4) besteht. Der Sohlenkörper (2) und die Fersenstütze (3) sind als einstückiges Kunststofformteil spritzgegossen. Die Oberseite (5) des Sohlenkörpers (2) weist eine Reihe von Vertiefungen - beispielhaft mit (6) bezeichnet - zur Materialersparnis auf. Die Unterseite (7) ist zur Verbeserung der Rutschfestigkeit geriffelt und teilweise gewölbt.

Der Sohlenkörper (2) weist einen vorderen Durchtritt (8) und einen hinteren Durchtritt (9) auf. Beide Durchtritte (8, 9) verlaufen quer durch den Sohlenkörper (2). Der vordere Durchtritt (8) besteht aus zwei Ösen (10, 11) und einer dazwischenliegenden Vertiefung (12) in der Oberseite (5). Der hintere Durchtritt (9) wird von vier Ösen (13, 14, 15, 16) gebildet, zwischen denen Vertiefungen (6) eingeformt sind.

Im Bereich des vorderen Durchtritts (8) stehen von den Seitenrändern (17, 18) in etwa halbkreisförmige Führungsstege (19, 20) hoch. Sie sind an dem Sohlenkörper (2) angeformt, wobei ihre Außenseiten mit den Seitenflächen des Sohlenkörpers (3) bündig sind. Die Führungsstege (19, 20) sind so dünn, ausgebildet, daß sie flexibel, jedoch nicht biegeschlaff sind. Unter Flexibilität ist in diesem Zusammenhang eine Biegsamkeit zu verstehen, bei der die Führungsstege (19, 20) einerseits ein seitliches Herausrutschen des Gehgipses noch zuverlässig verhindern, andererseits sich jedoch an die außenseitige Wölbung des Gehgipses anpassen können. Ihre maximale Höhe gegenüber den Seitenwandungen (17, 18) beträgt im Ausführungsbeispiel 3,5 cm und ihre Dicke an der Basis 3 mm. Letztere nimmt nach oben hin auf 2 mm ab.

In die beiden Durchtritte (8, 9) ist das einzige Gurtband (4) eingezogen, und zwar in der Weise, daß die aus den beiden Ösen (10, 11) des vorderen Durchtritts (8) austretenden Gurtbandabschnitte (22, 23) diagonal und sich kreuzend nach hinten verlaufen und jeweils auf der anderen Seite des Sohlenkörpers (2) in den Durchtritt (9) eintreten. Der obenseitig verlaufende Gurtbandabschnitt (22) besteht aus den Enden des Gurtbandes (21). Das eine Ende weist eine Klemmschnalle (24) auf, die von dem anderen Ende des Gurtbandes (21) durchsetzt wird. Die Festlegung dieses Endes geschieht in der Klemmschnalle (24) durch eine Exenterverklemmung. Dies ermöglicht eine stufenlose Anpassung der Längen der Gurtbandabschnitte (22, 23) an den jeweils mit der Gehgipssohle (1) zu versehenden Gehgips. Durch Strammziehen des freien Endes des Gurtbandes (21) legen sich die Gurtbandabschnitte (22, 23) fest um die Führungsstege (19, 20) und den Gehgips. Da sie beim Austritt aus den ösen (10, 11) des vorderen Durchtritts (8) über die Außenseite der flexiblen Führungsstege (19, 29) verlaufen, werden diese beim Strammziehen gegen den Gehgips herangebogen und passen sich an dessen Wölbung an. Dies geschieht auch dann, wenn der Gehgips eine etwas größere Breite hat als der Abstand der Führungsstege (19, 20).

Im vorderen Bereich ist in die Oberseite (5) des Sohlenkörpers (2) eine Quernut (25) eingeformt. Diese Quernut (25) erleichtert ein Abbrechen oder Absägen des davor liegenden Teils des Sohlenkörpers (2), wenn dies erwünscht ist.

## Ansprüche

1. Gehgipssohle mit einem Sohlenkörper, wobei in der Gehgipssohle wenigstens zwei Durchtritte zur Querführung eines darin eingezogenen Gurtbandsystems vorgesehen sind,
   dadurch gekennzeichnet, daß das Gurtbandsystem nur ein einziges Gurtband (4) aufweist, das die Durchtritte (8, 9) quer durchläuft, wobei jedoch die Gurtbandabschnitte (22, 23) außerhalb der Durchtritte (8, 9) und zwischen dem vorderen und hinteren Durchtritt (8, 9) diagonal und sich kreuzend von einer Seite zur anderen verlaufen und die beiden Enden außerhalb der Durchtritte (8, 9) über ein Verbindungsmittel (24) verstellbar gekuppelt sind.

2. Gehgipssohle nach Anspruch (1),
   dadurch gekennzeichnet, daß das Verbindungsmittel als Klemmschnalle (24) ausgebildet ist.

3. Gehgipssohle nach Anspruch (1),
   dadurch gekennzeichnet, daß das Verbindungsmittel als Klettverschluß ausgebildet ist.

4. Gehgipssohle nach einem der Ansprüche (1) bis (3),
   dadurch gekennzeichnet, daß die Gehgipssohle (1) hintenseitig eine nach oben vorstehende Fersenstütze (3) aufweist.

5. Gehgipssohle nach Anspruch (4),
   dadurch gekennzeichnet, daß der hintere Durchtritt (9) vor der Fersenstütze (3) angeordnet ist.

6. Gehgipssohle nach einem der Ansprüche (1) bis (5),
   dadurch gekennzeichnet, daß die Gehgipssohle (1) vorragende Seitenränder (17, 18) aufweist.

7. Gehgipssohle nach einem der Ansprüche (1) bis (6),
   dadurch gekennzeichnet, daß von den Seitenrändern (17, 18) des Sohlenkörpers (2) im Bereich des vorderen Durchtritts (8) Führungsstege (19, 20) hochstehen, die flexibel ausgebildet sind und über dessen Außenseiten das Gurtband (21) verläuft.

8. Gehgipssohle nach Anspruch (7),
   dadurch gekennzeichnet, daß die Höhe der Führungsstege (19, 20) gegenüber den Seitenrändern (17, 18) wenigstens 2,5 cm beträgt.

9. Gehgipssohle nach Anspruch (7) oder (8),
   dadurch gekennzeichnet, daß die Führungsstege (19, 20) an dem Sohlenkörper (2) angeformt sind.

10. Gehgipssohle nach einem der Ansprüche (7) bis (9),
    dadurch gekennzeichnet, daß die Erstreckung der Führungsstege (19, 20) in Längsrichtung der Gehgipssohle (1) nach oben hin abnimmt.

11. Gehgipssohle nach Anspruch (10),
    dadurch gekennzeichnet, daß die Führungsstege (19, 20) in etwa eine Halbkreisform haben.

12. Gehgipssohle nach einem der Ansprüche (7) bis (11),
    dadurch gekennzeichnet, daß die Dicke der Führungsstege (19, 20) nach oben hin abnimmt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | US-A-3 798 803 (KENNEDY)<br>* Figuren 1-3; Anspruch 1 * | 1 | A 61 F 13/04 |
| A | | 4,6 | |
| Y | DE-U-1 888 536 (SAILER)<br>* Ansprüche 1,3; Figuren 1,2 * | 1 | |
| A | FR-A-6 863 25 (COUSTERE)<br>* Figuren 1,2; Seite 1, Zeile 59 - Seite 2, Zeile 11 * | 1 | |
| A | GB-A-2 147 792 (SLAUGHTER et al.)<br>* Ansprüche 1,5,7; Figur 1 * | 1,2 | |
| A | DE-C-5 934 79 (WELZ)<br>* Anspruch; Figuren 1,3 * | 1 | |
| D,A | DE-C-2 740 400 (BLOEMER)<br>* Figur 1; Anspruch 1 * | 1,6 | |

|  | RECHERCHIERTE SACHGEBIETE (Int. Cl.5) |
|---|---|
| | A 43 B<br>A 61 F |

Der vorliegende Recherchenbericht wurde für alle Patentansprüche erstellt

| Recherchenort | Abschlußdatum der Recherche | Prüfer |
|---|---|---|
| Berlin | 11 März 91 | KANAL P K |